# EUROPEAN PATENT APPLICATION

(11) **EP 4 338 800 A1**
(43) Date of publication of application: **20.03.2024**
(21) Application number: 22806224.6
(22) Date of filing: 08.02.2022
(51) Int. Cl.: A61P 35/00, A61K 47/46, A61K 45/00, A61K 9/16, C12N 7/01, C12N 5/10

(54) **USE OF CELLULAR MICROPARTICLES IN TREATMENT OF RESPIRATORY VIRAL PNEUMONIA**

(30) Priority: 12.05.2021 CN 202110517772
(71) Applicant: Institute of Basic Medical Sciences, Chinese Academy of Medical Sciences, Beijing 100005 (CN)
(72) Inventor: HUANG, Bo, Beijing 100005 (CN)
(74) Representative: Sagittarius IP
(86) International application number: PCT/CN2022/075503
(87) International publication number: WO 2022/237247

(57) **Abstract**

Disclosed is the use of cellular microparticles in the treatment of respiratory viral pneumonia. Tumor-cell-derived microparticles (Nws), which comprise a cellular vesicle released by an apoptotic tumor cell. The cellular vesicle expresses a spike protein binding receptor on the surface thereof. The MPs have high biosafety. In addition, it is found that the receptor on the surface of the microparticle has an ability to adsorb a virus, and macrophages can efficiently ingest the MPs, which can be used for treating viral pneumonia.

## Description

### FIELD OF THE INVENTION

The present application relates to biological, medical and clinical fields. Specifically, the present application relates to use of cellular microparticles in the treatment of Coronavirus Disease 2019 infection.

### BACKGROUND OF THE INVENTION

Viral pneumonia can outbreak or can become sporadic and endemic. Viral pneumonia can occur at any time of the year, but is more common in winter and spring. Initially, viral pneumonia is predominantly a viral infection of the upper respiratory tract, causing pneumonia as the virus spreads downward to lung.

Viral pneumonia can be transmitted by droplets. The clinical manifestations are generally mild, mainly symptoms similar to respiratory diseases, such as headache, fatigue, fever and cough. Respiratory tract infection is one of the leading causes of death in the world, especially patients with severe pneumonia have high mortality and serious sequelae. At present, viruses such as influenza virus and coronavirus are the main pathogens that cause regional outbreak of severe pneumonia, are highly contagious and have a high fatality rate.

Coronavirus Disease 2019 (COVID-19) is an acute respiratory infectious disease caused by 2019 novel coronavirus (SARS-CoV-2).

SARS-CoV-2 is a novel strain of coronavirus that has never been found in humans before, and the main transmission routes are respiratory droplet transmission and contact transmission. COVID-19 is mainly manifested as fever, dry cough and fatigue, and a small number of patients also have nasal congestion, runny nose, diarrhea and other upper respiratory and gastrointestinal symptoms. Patients with severe illness usually have difficulty in breathing in about one week, and in severe cases it rapidly progress to acute respiratory distress syndrome, septic shock, coagulation dysfunction, multiple organ failure, etc., which eventually lead to the death of the patient. SARS-CoV-2 is highly contagious and has a high fatality rate.

After the outbreak of pneumonia caused by 2SARS-CoV-2, despite great efforts in drug development in various countries, so far, there are still no effective drugs for treating SARS-CoV-2 infection. For the prevention and treatment of COVID-19, vaccine research and development has made great progress, and China has issued marketing approval for 4 COVID-19 vaccines, including three inactivated vaccines and one adenovirus vector vaccine. Although vaccines can greatly prevent the spread of the virus, there is still a need to explore more effective therapeutic regimens for patients already infected by the virus. At present, drug development for COVID-19 is mainly to screen small molecule compounds against viral replication and packaging, but many small molecule compounds face the challenges of uncertainty and safety issues. Although many drugs exhibit good anti-coronavirus activity *in vitro,* they often do not have the value of *in vivo* application in humans. Some drugs have been used in clinical antiviral treatments, but these drugs are not specific for SARS-CoV-2, and their side effects cannot be ignored.

Therefore, it is necessary to explore unconventional treatment strategies for patients with COVID-19.

### SUMMARY OF THE INVENTION

The present application provides a tumor cell-derived microparticle derived from a cellular vesicle of an apoptotic tumor cell, which expresses a spike protein binding receptor thereon and acts as a targeting agent, more easier access to the pneumonia treatment site.

Those skilled in the art know that a cell constitutes of a cell membrane that enclose the cell contents and the cell membrane consists of protein molecules embedded in phospholipid bilayers, and that the spherical structure of the cell is maintained by the traction force formed by protein fiber filaments called the cytoskeleton. When the cell is stimulated by foreign signals (e.g., chemotherapy drugs, ultraviolet rays, radiation, etc.) and undergoes apoptosis, the protein fiber filaments are broken or lose attachment, and the traction force disappears, so that the local cell membrane structure expands outward, protrudes and wraps the cell contents in the form of vesicles. The vesicle has a size of about 100 to 1000 nanometers, and is the "cellular vesicle" described in the present application.

The above-mentioned cellular vesicle is used as a carrier to express targeting agents thereon, which is more conducive to targeting the therapeutic target. This type of vesicle cannot enter normal tissues (with a permeability of about 5 to 10 nm), does not cause damage to normal tissues, and thus avoids the side effects caused by exogenous carriers such as nanomaterials.

In some embodiments, the cellular vesicle is derived from a tumor cell, especially the tumor cell is derived from same type of tissue as that of the site to be treated. In some embodiments, the cellular vesicle can be easily contacted with the cell membrane of the site to be treated in the patient's body. In some particular embodiments, the site to be treated in the patient is the lung. In some embodiments, the tumor cell is a lung cancer cell; thus, the cellular vesicle is derived from a lung cancer cell.

In other embodiments, the tumor cell type used to prepare the cellular vesicle can be different from the cell type at the site where the cellular vesicle is to be administered. As an example, the cellular vesicle of the present application is prepared from colon cancer cells, however, the cellular vesicle is allowed to be administered to lung.

In a particular embodiment, the tumor cell used to prepare the cellular vesicle contains oxysterols, such as cholesterols containing hydroxylation modifications. As an example, the tumor cell used to prepare the cellular vesicle contains 25-hydroxylated cholesterol.

There are many well-known available human tumor cell lines in the art, and their oxysterol contents are different. In some embodiments, tumor cell lines rich in oxysterols (such as hydroxylated cholesterols) are allowed to be selected as a source for the preparation of the cellular vesicle.

In some embodiments, the tumor cell is a lung cancer cell, for example, but not limited to NCI-H196, NCI-H292, NCI-H460, NCI-H446, NCI-H1299, NCI-H1650, H1792, NCI-H3255, A427, A549, 0225-02Sp, 2F7, 95-D, SPCA-1, LLC, Calu-1, Calu-3, L1022, PC9R, MSTO-211H, TKB-1. In one particular embodiment, the tumor cell is Calu-3 rich in oxysterols. In one particular embodiment, the tumor cell is A549 rich in oxysterols.

In some embodiments, the tumor cell is a colon cancer cell line. The colon cancer cell is selected from the group consisting of HCT116, HCT116/FU, HCT-8/FU, LoVo, LoVo ADR, SW480, SW620, CaCo-2, RKO-E6, RKO-AS45-1, FET, HT55, HT115, HT-29, COLO 205, KM12, CL-40, KM12-SM, COLO320DM, NCI-H508, SW1417, COLO394 and WiDr. In one particular embodiment, the tumor cell is Caco-2 rich in oxysterols.

In some embodiments, the targeting agent is a spike protein binding receptor expressed on the surface of the vesicle.

In some embodiments, the spike protein binding receptor expressed on the cellular vesicle can be naturally expressed or recombinantly expressed.

In some embodiments, the spike protein is a pneumonia virus spike protein (S; Spike).

In some embodiments, the virus is selected from the group consisting of SARS-CoV, SARS-CoV-2 and variants thereof.

In some particular embodiments, the spike protein binding receptor is angiotensin-converting enzyme 2 or a binding fragment thereof.

In some particular embodiments, the ACE2 is human ACE2. The term encompasses naturally occurring human ACE2 or naturally occurring variants thereof, as well as artificially expressed human ACE2 or variants thereof, such as recombinant human ACE2 expressed *in vitro* or variants thereof.

The "binding fragment of angiotensin-converting enzyme 2" described in the present application refers to a fragment of angiotensin-converting enzyme 2, as long as such fragments still retain their ability to specifically bind to the spike protein. The binding fragment of angiotensin-converting enzyme 2 also fall within the scope of the present application. The angiotensin-converting enzyme 2 or binding fragments thereof in the present application can be naturally expressed, recombinantly expressed, or genetically engineered.

In some embodiments, the tumor cell is selected from a lung cancer cell and a colon cancer cell.

The present application provides a method for preparing a tumor cell-derived microparticle, by subjecting the tumor cell to apoptosis so as to obtain the cellular vesicle, by using any feasible means.

In some particular embodiments, provided is a method for preparing a tumor cell-derived microparticle, comprising the following steps:
1) providing a tumor cell expressing a spike protein binding receptor;
2) subjecting the tumor cell of step 1) to apoptosis;
3) collecting the cellular vesicle released by the apoptotic tumor cell.

In some particular embodiments, the cell is subjected to apoptosis by any well-known means in the art. Non-limiting examples include ultraviolet irradiation, X-ray irradiation, or chemotherapy drugs such as dexamethasone. In the method of the present application, apoptosis can be performed without introducing exogenous substances to the tumor cell, therefore, radiation is preferred (e.g., ultraviolet irradiation, X-ray irradiation). In some particular embodiments, the period of radiation and radiation intensity can be determined by those skilled in the art according to routine operations.

In particular embodiments of the present application, preferably, ultraviolet ray or chemotherapy drugs are used to induce apoptosis of the tumor cell. For the collection of the cellular vesicles, isolation can be performed by using ultracentrifuge under low temperature conditions (or room temperature conditions). Preferably, the cellular vesicle is collected by a centrifuge under low temperature conditions (such as around 4°C) with a centrifugal force of 100 to 100,000 g.

In some particular embodiments, the cellular vesicle released by the apoptotic tumor cell is collected by centrifugation, the average particle size of the cellular vesicle is 200 nm to 800 nm, preferably 300 nm, 400 nm, 500 nm, 600 nm, 700 nm, 800 nm ± 10%.

In some particular embodiments, provided is a method for preparing a tumor cell-derived microparticle, comprising the following steps:
1) providing a human tumor cell expressing human ACE2 or a binding fragment thereof, the human tumor cell is a lung cancer cell or a colon cancer cell;
2) subjecting the tumor cell of step 1) to apoptosis;
3) collecting the cellular vesicle released by the apoptotic tumor cell by a means comprising the following steps:
   centrifuging the apoptotic tumor cell obtained in step 2) at 2 to 8°C at 800 to 1,000 g for 5 to 15 minutes to remove intact cell and obtain a first product;
   centrifuging the first product at 2 to 8°C at 12,000 to 15,000 g for 1 to 3 minutes to remove cell debris and obtain a second product;
   centrifuging the second product at 2 to 8°C at 12,000 to 15,000 g for 40 to 80 minutes to collect the released cell vesicle.

In some particular embodiments, the cellular vesicle released by the apoptotic tumor cell is collected by a means comprising the following steps:
centrifuging the obtained apoptotic tumor cell at 2 to 8°C at 1,000 g for 10 minutes to remove intact cell and obtain a first product;
centrifuging the first product at 2 to 8°C at 14,000 g for 2 minutes to remove cell debris and obtain a second product;
centrifuging the second product at 2 to 8°C at 14,000 g for 60 minutes to collect the released cell vesicle; the average particle size of the cellular vesicle collected according to these steps above fall into the range of 100 nm to 1,000 nm, especially 200 to 800 nm.

The present application provides a tumor cell-derived microparticle prepared by the aforementioned method.

The present application provides a pharmaceutical composition comprising the tumor cell-derived microparticle according to the present application.

According to preferred embodiments of the pharmaceutical composition of the present application, the pharmaceutical composition comprises the cellular vesicle or the tumor cell-derived microparticle.

For the collected microparticle, it can be prepared into a pharmaceutical composition, especially a nebulized formulation or a spray formulation , according to conventional means.

As preferred embodiments of the present application, the average particle size in the pharmaceutical composition formed by the tumor cell-derived microparticle is 100 to 1000 nanometers, and non-limiting examples that may be mentioned are 100, 150, 200, 250, 300, 350, 400, 450, 500, 550, 600, 650, 700, 750, 800, 900, 950, 1000 ± 10% nm.

The drug composition provided by the present application can be administered in accordance with conventional clinical treatment methods, for example, for pneumonia, it can be administered directly by nasal drops, sprays, or perfusion into the lungs; the dose used for administration can be the dose determined by the healthcare practitioner.

In particular embodiments, the microparticle, cellular vesicle or pharmaceutical composition provided by present application is prepared into a dosage form suitable for lung administration.

As an example, inhalable powders for lung administration can be produced by conventional techniques such as jet milling, spray drying, solvent precipitation, supercritical fluid condensation, etc. Metered dose inhalers produced by using dry powder inhalers (DPIs), such as those based on Nektar^{™}, Vectura (Gyrohaler^{™}) and GSK (Discus^{™}) or Astra (Turbohaler^{™}), etc., comprise the microparticle, cellular vesicle or pharmaceutical composition of the present application in a suitable carrier (such as mannitol, sucrose or lactose), which are delivered to the surface of the terminal alveoli. An ultrasonic nebulizer can also be used to deliver a solution preparation with liposomes (or without liposomes) to lung.

According to some embodiments of the present application, provided is a A549 cell-derived microparticle, which can be used as a therapeutic means for a patients with a COVID-19 infection. Angiotensin-converting enzyme 2 (ACE2) present on the surface of the microparticle is a receptor capable of binding to a SARS-CoV-2 surface protein and mediating viral entry. Therefore, MPs can adsorb SARS-CoV-2 virus and limit the spread of the virus in the body. Virus-carrying microparticles are efficiently taken up by macrophages and delivered to lysosomes for degradation, thereby enabling effective treatment of COVID-19.

In the present application, the term "microparticle (NW)" refers to a vesicle-like structure that sheds from the surface of eukaryotic cell membrane upon activation or apoptosis, with an average particle size of 100 to 1,000 nm. The microparticle is considered to be a carrier of bioinformation, mediating the transmission and exchange of bioinformatic substances between different types of cells. Due to the characteristics such as high biocompatibility, low immunogenicity and targeting ability, MP is served as a carrier for drugs, the tumor cell-derived microparticle loaded with an anti-tumor drug has good anti-tumor effect and has been applied in clinical practice.

In some embodiments, the microparticle formulation is administered in an animal experiment at a dose of 5×10⁶/50 µl, once daily for 5 days. Those skilled in the art can determine the unit dose of human subjects based on the animal experiments.

### DESCRIPTION OF THE DRAWINGS

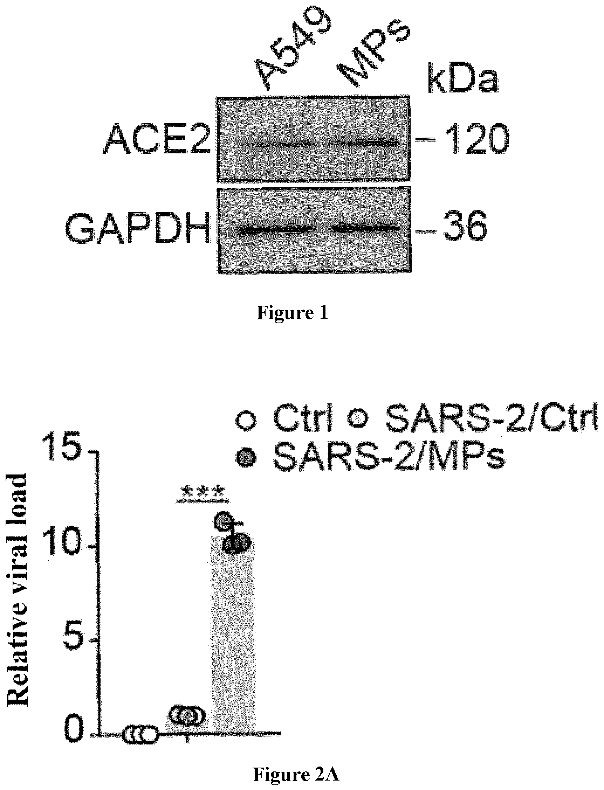

Figure 1 shows ACE2 expression in A549 cell-derived microparticles (Ml's), and Western blot analysis demonstrates ACE2 expression in MPs.

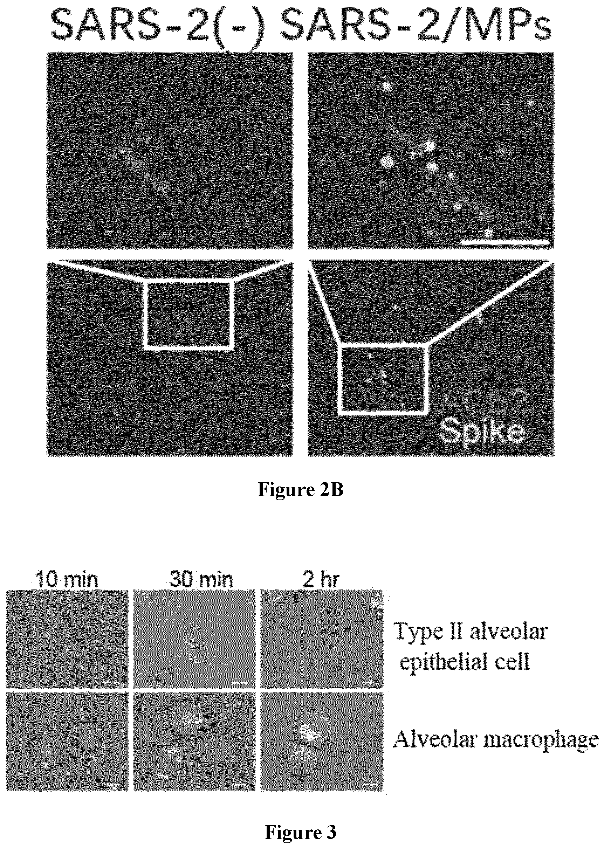

Figures 2A and 2B show that SARS-CoV-2 can be adsorbed by MPs. After incubating AMPs with SARS-CoV-2, the solution is filtered through a 0.1 Mp type filter, and real-time PCR analysis shows that viral RNA can be detected in incubated MPs and not in unincubated MPs (Figure 2A). Immunofluorescent staining further confirms binding of the viral S protein to ACE2 on the MPs (Figure 2B).
Figure 3 shows that MPs adsorb virions and deliver them to alveolar macrophages. *In vitro* incubation of alveolar macrophages with virus-adsorbing MPs shows that macrophages can take up virus-containing MPs within 10 minutes, while isolated primary type II alveolar epithelial cells hardly take up MPs.

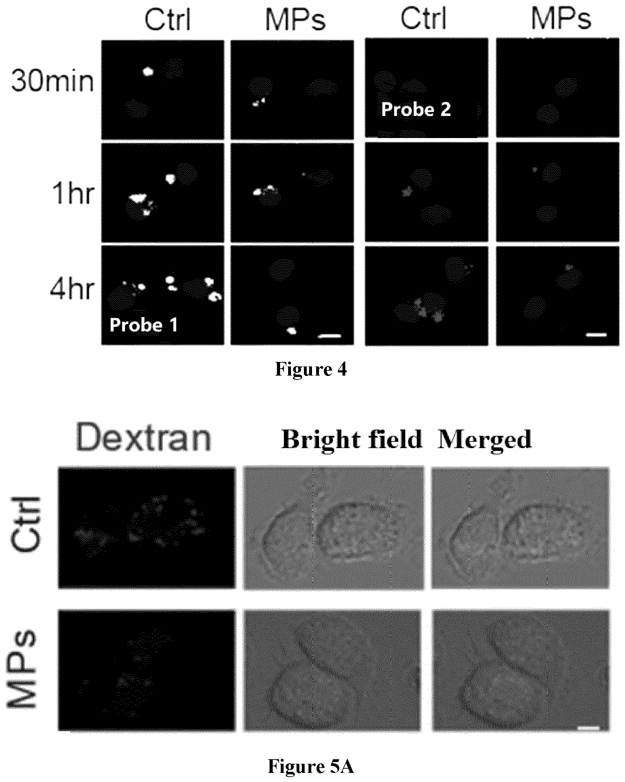

Figure 4 shows that the SARS-CoV-2 virus adsorbed by MPs is inactivated in alveolar macrophages. After incubating SARS-CoV-2, SARS-CoV-2/MPs with alveolar macrophages for 0.5, 1 and 4 hours, SARS-CoV-2 can replicate in alveolar macrophages, and SARS-CoV-2 bound to MPs has a reduced load in alveolar macrophages. Probe 1 targets the viral sense sequence to assess viral distribution (green), and Probe 2 targets the viral antisense sequence to indicate viral replication (red) (according to conventional design principles, those skilled in the art are able to design probes).
Figure 5A shows a confocal microscope image with a scale bar of 5 µm.

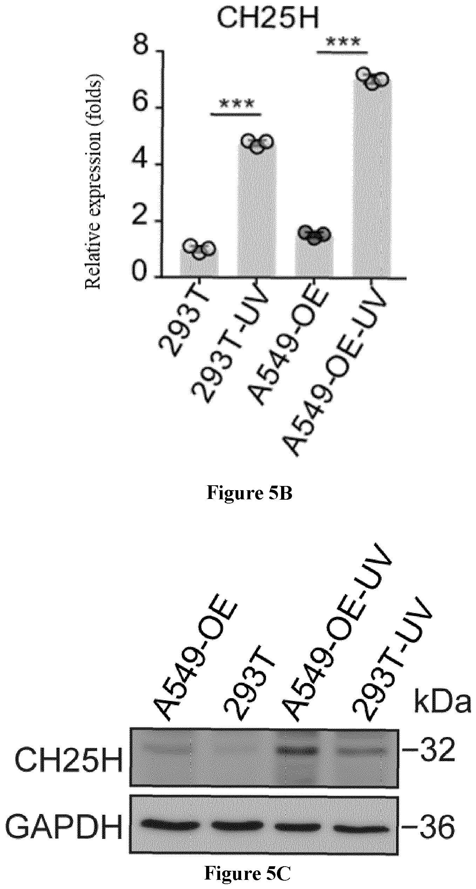

Figures 5B to 5D show that the cholesterol-25-hydroxylase in irradiated A549-ACE2-OE cells is significantly upregulated, and the content of 25-hydroxycholesterol of the extracted microparticles is significantly increased. Note: 293T represents the human renal epithelial 293 cell line (a cell line with low hydroxylated cholesterol content); 293T-UV represents 293T cells after 1 h of ultraviolet irradiation; A549-OE represents A549 ACE2-overexpressing cells; A549-OE-UV represents A549 ACE2-overexpressing cells after 1 h of ultraviolet irradiation; 293-MPs represents 293T cell-derived microparticles; AO-Ml's represents A549-ACE2-overexpressing cell-derived microparticles.

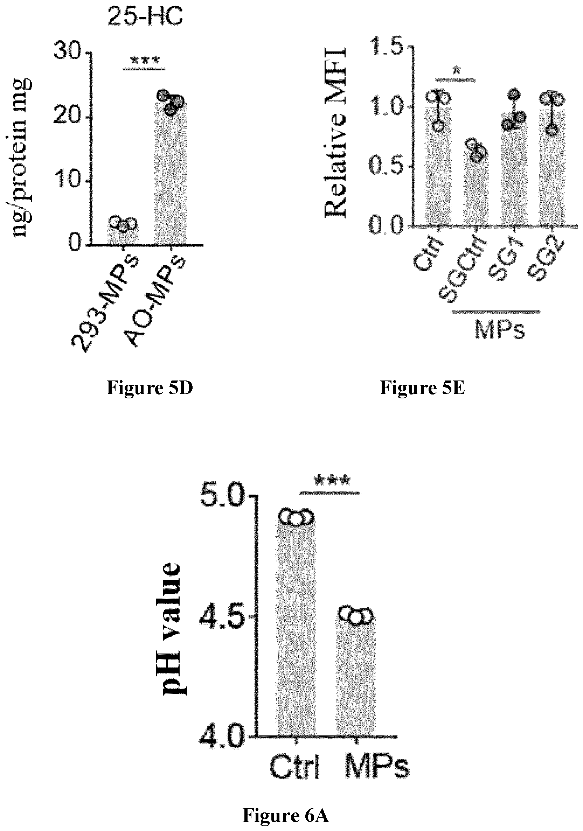

Figure 5E shows that by knocking out cholesterol-25-hydroxylase (CH25H for short), the effect of microparticles upregulating endosomal pH disappears. Ctrl represents the PBS-only group; SGCtrl represents the group with A549-derived control microparticles; SG1 represents the group with A549-CH25H-knockout cell-derived microparticles; SG2 represents the group with A549-CH25H-knockout cell-derived microparticles.

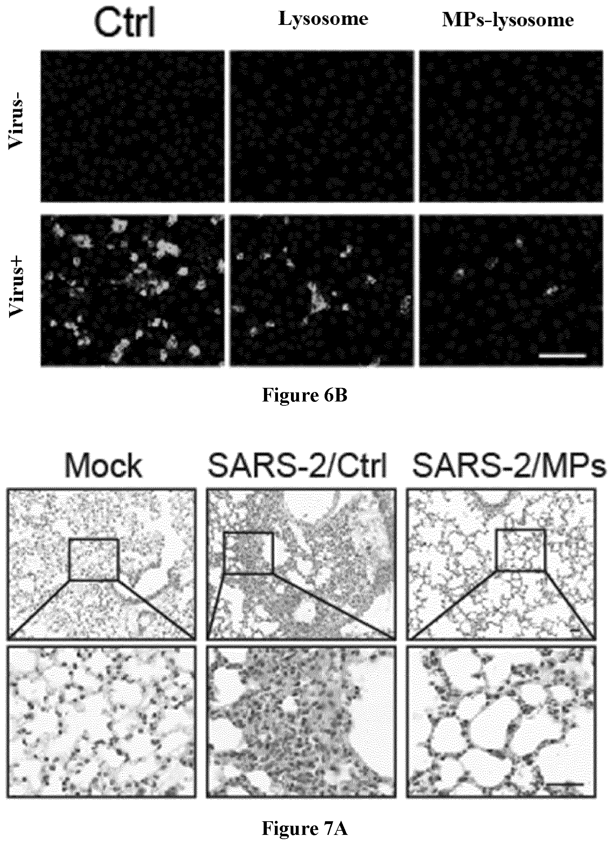

Figures 6A and 6B show that MPs enhance the degradation of SARS-CoV-2 virus by reducing the pH in lysosomes. Treating alveolar macrophages with MPs results in a decrease in lysosomal pH (Figure 6A), and lysosomes isolated from NWs-treated macrophages inactivate the virus more efficiently than lysosomes isolated from control macrophages (Figure 6B).

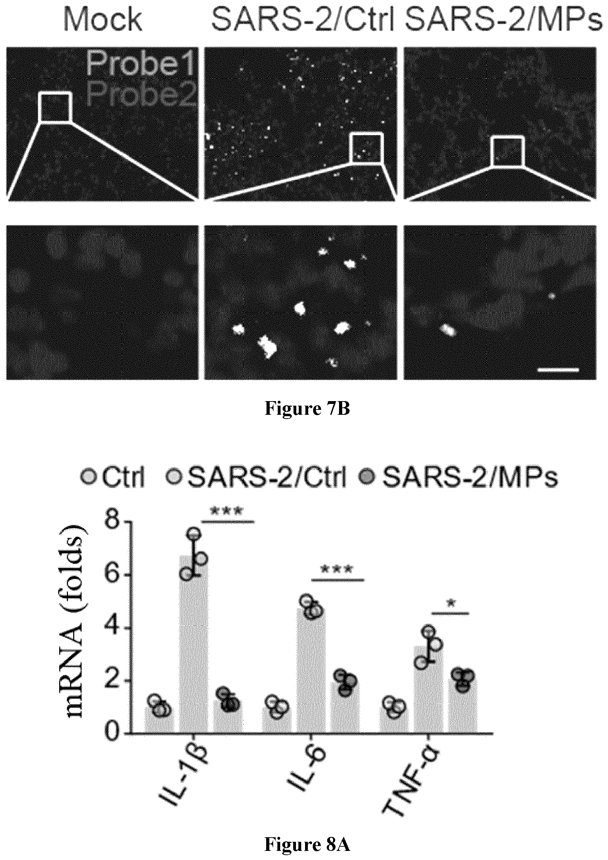

Figures 7A to 7B show the *in vivo* efficacy of MPs in treating SARS-CoV-2 virus infections. After 5 days of SARS-CoV-2 infection, mice are injected with MPs into the nasal cavity for 5 consecutive days. H&E staining shows a decrease in peribronchial and perivascular inflammatory cell infiltration and a reduction in lung histopathological damage in mice in the treatment group (Figure 7A). Consistent with the improvement in morphology, RNA Scope shows a decrease in viral load in the lungs (Figure 7B). Ctrl: control; Mock: PBS treated group.
Figure 8A shows the levels of TNF-α, IL-1β and IL-6 inflammatory factors in *in vitro* macrophages that have taken up SARS-CoV-2-carrying MPs, compared to those that have taken up SARS-CoV-2 alone.

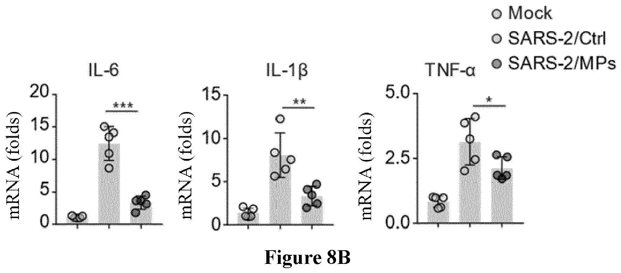

Figure 8B shows the levels of TNF-α, IL-1β and IL-6 inflammatory factors in lung tissues of Ml's-treated mouse. Note: Ctrl: control; Mock: PBS treated group.

### DETAILED DESCRIPTION OF THE INVENTION

Various cell lines, agents and experimental animals used in the examples:
The mouse macrophage cell line Raw264.7 and A549 human lung adenocarcinoma cell line were purchased from China Center for Type Culture Collection (CCTCC);
Female ICR, hACE2 transgenic mice, 6 to 8 weeks old, were purchased from the Medical Laboratory Animal Center, Chinese Academy of Medical Sciences (Beijing). The study of mice without virus infection was approved by the Animal Protection and Utilization Committee, Chinese Academy of Medical Sciences.

### Example 1. ACE2 is expressed in A549 cell-derived microparticles (MPs)

### 1. Experimental steps

The human ACE2 coding sequence was amplified and inserted into the plasmid pLV-EF1α-IRES-Puro, which was transiently expressed in 293T cells to obtain a virus containing the human ACE2 gene. The human ACE2-containing lentivirus was transduced into A549 cells, which were then screened with 1 µg/ml puromycin to obtain cell clones with high ACE2 expression, namely A549-ACE2-OE.

In order to construct a stable knockout ACE2 cell line, RNA for knockout was designed for the well-known human ACE2 gene sequence in the database. The RNA was cloned into the pSpCas9(BB)-2A-GFP vector plasmid, which was used to transfect cells. After 48 hours, GFP-positive cells were sorted by flow cytometry using BD Biosciences FACSAria III. Candidate knockout cells were validated by Western blot or immunofluorescence to obtain A549-ACE2-SG (ACE2-knockout A549 cells).

A549, A549-ACE2-OE, and A549-ACE2-SG were each irradiated with 300 J/m² ultraviolet ray for 1.5 hours, and the supernatant was collected after 18 hours. The supernatant was centrifuged at 1,000 × g for 10 min to remove cells, followed by centrifugation at 14,000 × g for 2 min to remove debris. Then, the supernatant was centrifuged at 4°C at 14,000 × g for 60 min to prepare each type of MPs. The MPs were washed 3 times and suspended in culture medium to perform subsequent experiments.

A549 cells and each type of MPs were lysed in lysis buffer and sonicated. The protein concentration was determined by BCA kit. Then the proteins were resolved on SDS-PAGE gels and transferred onto nitrocellulose membranes. The nitrocellulose membrane was blocked with 5% bovine serum albumin and detected overnight with antibodies to detect the expression of ACE2 in MPs, with A549 cells as a positive control.

### 2. Experimental results

Western blot analysis showed the expression of ACE2 in MPs and the presence of ACE2 in A549-derived microparticles obtained using this method (Figure 1).

### Example 2. SARSCoV-2 virus can be adsorbed by MPs

### 1. Experimental steps

The MPs obtained from A549-ACE2-OE were incubated with SARSCoV-2 virus at 37°C for 30 min and then filtered through a 0.1 µm filter membrane. The filter could selectively allow virus particles to pass through, but prevent MPs from passing through. The SARS CoV-2 virus that was not incubated with MPs was used as a control group and was also filtered through a filter membrane. The viral load on the membranes of different groups was detected by real-time quantitative PCR.

MPs (5×10⁵) were incubated with recombinant SARS-CoV-2 spike protein (0.1 µg), fixed, and then stained with anti-ACE2 protein (red) and anti-spike protein (green) antibodies. Fluorescence was determined by super-resolution structured illumination microscopy, with a scale bar of 2 µm.

### 2. Experimental results

After incubating AMPs with SARS-CoV-2, Q-PCR analysis showed that viral RNA could be detected in incubated MPs but not in unincubated MPs (Figure 2A). Immunofluorescent staining results confirmed binding of the viral S protein to ACE2 on the MPs (Figure 2B).

### Example 3. MPs adsorb virions and deliver them to alveolar macrophages

### 1. Experimental steps:

Primary alveolar macrophages were isolated from mouse bronchoalveolar lavage fluid. Mice were anesthetized immediately before lavage, and the trachea was dissected. Lungs were laved 5 times with 1 ml PBS and the lavage fluid was centrifuged at 4°C at 600 × g for 5 min. The collected cells were suspended in RPMI1640 complete medium, cultured on culture plates for 2 h, and then gently washed with PBS to remove non-adherent cells.

Primary alveolar epithelial cells were isolated from hACE2 mice perfused with 10 milliliters of cold PBS through the right ventricle. The lung tissue was filled with 2 ml racemase and low gelling temperature agarose gel, and then incubated with 2 ml racemase at 37°C for 20 min. The lung tissue was then grounded and the homogenate was filtered through 70 and 40 µm nylon meshes. Biotin-labeled antibodies and magnetic beads were used to exclude leukocytes, monocytes/macrophages, NK cells, neutrophils, endothelial cells, and erythroid cells from the cell suspension.

Primary alveolar macrophages and primary alveolar epithelial cells of ICR mice were isolated. After treatment with PKH67-labeled A-OE-Ml's for 10 min, 30 min and 2 h, images were captured under a confocal microscope with a scale bar of 5 µm.

### 2. Experimental results:

*In vitro* incubation of alveolar macrophages with virus-adsorbing MPs showed that macrophages could take up virus-containing MPs within 10 minutes, while isolated primary type II alveolar epithelial cells could hardly take up MPs even in 2 hours (Figure 3).

### Example 4. The SARS-CoV-2 virus adsorbed by MPs is inactivated in alveolar macrophages

### 1. Experimental steps:

MPs (5×10⁵) were incubated with SARS-CoV-2 (5 × 10⁴ TCID₅₀) at 37°C for 30 min, and then alveolar macrophages were added for treatment for 30 min, 1 h and 4 h. MPs unincubated with SARS-CoV-2 were used as a control group. Cells were fixed in 4% paraformaldehyde, incubated with hydrogen peroxide at room temperature for 10 min, and RNA *in situ* analysis was performed using RNAScope kits. Probe 1 targeted the viral sense sequence to assess viral distribution (green), and Probe 2 targeted the viral antisense sequence to indicate viral replication (red), with a scale bar of 5 µm (according to conventional design principles, those skilled in the art are able to design Probes 1 and 2).

### 2. Experimental results:

After incubating SARS-CoV-2, SARS-CoV-2/MPs with alveolar macrophages for 0.5, 1 and 4 hours, SARS-CoV-2 alone could replicate in alveolar macrophages, and SARS-CoV-2 bound to MPs had a reduced load in alveolar macrophages (Figure 4).

### Example 5. MPs prevent viral evasion by increasing endosomal pH

Macrophages were treated with microparticles for 30 min in advance, then pHrodo^{™} Red dextran was added for labeling for 10 min and a confocal microscope was used for imaging. Scale bar 5 µm.

A549-ACE2-OE cells were seeded in six-well plates (5×10⁵) and subjected to ultraviolet irradiation at 300 J/m² for 1 hour. RNA was collected after 18 h to detect CH25H content by qPCR. At the same time, this method was used to extract microparticles, and the content of 25-hydroxycholesterol (25HC) in MPs was detected by mass spectrometry experiments. CH25H in A549 cells was knocked out using the CRISPR-Cas9 system, followed by extraction of MPs, which were used to treat macrophages for 30 min. Then pHrodo^{™} Red dextran was added for labeling, and a confocal microscope was used for imaging (Figure 5A).

The results showed that the microparticles could increase the pH of endosomes, and inhibit the viral replication by preventing virus from escaping to cytoplasm. After being treated with microparticles, the endosomal pH of macrophages was increased. Oxysterols carried by microparticles affected the pH of endosomes. Figures 5B, 5C and 5D showed that the cholesterol-25-hydroxylase in irradiated A549-ACE2-OE cells was significantly upregulated, and the content of 25-hydroxycholesterol in the extracted microparticles was also significantly increased. Figure 5E showed that by knocking out CH25H, the effect of microparticles upregulating endosomal pH disappeared.

### Example 6. MPs enhance the degradation of SARS-CoV-2 virus by reducing the pH in lysosomes

### 1. Experimental steps

Alveolar macrophages were pre-treated with MPs obtained from A549-ACE2-OE for 30 min, and alveolar macrophages not treated with MPs were used as a control group. The macrophages were stained with LysoSensor^{™} Yellow/Blue DND-160, the pH value was detected with a microplate reader, and the pH of macrophage lysosomes was determined by LysoSensor^{™} Yellow/Blue DND-160 using a lysosomal pH meter. The results showed that the pH of living cells depends on two-photon excitation spectroscopy.

Lysosomes were isolated and purified, and adherent cells were digested by trypsin and then washed with ice-cold PBS. The cell pellet was resuspended and placed into a homogenizer for disruption. The cell homogenate was centrifuged at 1,000 × g for 10 min and the supernatant was centrifuged at 20,000 × g for 20 min to prepare lysosomal particles and other organelles. The top (lowest density) band was removed by establishing a density gradient and centrifuging in a SW50.1 rotor at 150,000 × g for 4 h, and diluted in PBS. After washing, the lysosomes were centrifuged at 20,000 ×g for 20 min for isolation and purification.

Lysosomes isolated from MPs-treated macrophages and lysosomes isolated from control macrophages were incubated respectively with SARS-CoV-2 at 37°C for 30 min, and then virus E6 was added for infection for 48 h. The cells were stained with anti-NP antibodies. Scale bar 5 µm.

### 2. Experimental results

Treating alveolar macrophages with MPs resulted in a decrease in lysosomal pH (Figure 6A), and lysosomes isolated from MPs-treated macrophages had lower viral load than lysosomes isolated from control macrophages (Figure 6B).

### Example 7. In vivo efficacy of MPs in treating SARS-CoV-2 virus infections

### 1. Experimental steps

hACE2 mice were infected intratracheally with SARS-CoV-2 (1×10⁵ TCID₅₀) and then treated with a control group (PBS) or MPs (5×10⁶). The treatment was given once a day for 5 consecutive days (n = 5 animals/group). After 5 d of treatment, the mice were sacrificed, and the lung tissues were fixed for HE staining.

After fixing of the lung tissue, RNA *in situ* analysis was performed using RNAScope kits. Probe 1 targeted the viral sense sequence to assess viral distribution (green), and Probe 2 targeted the viral antisense sequence to indicate viral replication (red), with a scale bar of 5 µm.

### 2. Experimental results

H&E staining showed that the peribronchial and perivascular inflammatory cell infiltration in mice in the treatment group was reduced, and the pathological damage of lung tissue was alleviated (Figure 7A). Consistent with the improvement in morphology, RNAScope showed a decrease in viral load in the lungs (Figure 7B).

### Example 8. Macrophages do not cause an inflammatory response when eliminating MPs-adsorbed SARS-CoV-2

SARS-CoV-2 (1×10⁵ TCID₅₀) was added to macrophages (1×10⁵) for 24 h. The RNA was extracted by TRIZOL method, and the levels of TNF-α, IL-1β and IL-6 were detected by qPCR.

hACE2 mice were infected intratracheally with SARS-CoV-2 (1×10⁵ TCID₅₀) and then treated with a control group (PBS) or MPs (5×10⁶), respectively. The treatment was given once a day for 5 consecutive days (n=5 animals/group). Mice were sacrificed after 5 days of treatment. The lung tissue RNA was extracted by TRIZOL method, and the levels of TNF-α, IL-1β and IL-6 was detected by qPCR.

Figure 8A showed that the levels of TNF-α, IL-1β and IL-6 inflammatory factors in *in vitro* macrophages that have taken up SARS-CoV-2-carrying MPs were significantly decrease, compared to those that have taken up SARS-CoV-2 alone. Figure 8B showed that the levels of TNF-α, IL-1β and IL-6 inflammatory factors in MPs-treated mouse lung tissues were significantly down-regulated.

In summary, the technical solutions of the present application have the following effects:
1. The A549 cell-derived microparticles (MPs) provided in the present application are used to treat SARS-CoV-2 infections, utilizing its characteristics of adsorbing SARS-CoV-2 virus and enhancing the ability of alveolar macrophages to clear SARS-CoV-2, as a novel unconventional treatment strategy.
2. After viral infection, by intratracheal injection of MPs, the ACE2 present on the surface of the microparticles can bind to the surface S protein of SARS-CoV-2 and adsorb the virus particles, thereby limiting the further spread of the virus in body. The virus-carrying microparticles will be efficiently taken up by macrophages and delivered to lysosomes. MPs enhances the ability of macrophages to eliminate SARS-CoV-2 by adjusting the lysosomal pH value, so as to achieve clinical treatment of COVID-19. MPs can thereby effectively reduce the mortality rate of COVID-19 patients, and have good clinical application prospects.
3. The microparticles of the present application are derived from cells, with characteristics such as high biocompatibility, low immunogenicity, targeting ability, etc., so they have high safety and no toxicity or side effects.

## Claims

1. A tumor cell-derived microparticle, comprising a cellular vesicle released by an apoptotic tumor cell, the cellular vesicle expresses a spike protein binding receptor on the surface thereof;
preferably, the spike protein is a pneumonia virus spike protein;
more preferably, the pneumonia virus is selected from the group consisting of SARS-CoV, SARS-CoV-2, and variants thereof;
most preferably, the spike protein binding receptor is angiotensin-converting enzyme 2 (ACE2) or a binding fragment thereof;
the tumor cell is a lung cancer cell or a colon cancer cell.

2. The tumor cell-derived microparticle according to claim 1, wherein:
preferably, the lung cancer cell is selected from the group consisting of NCI-H196, NCI-H292, NCI-H460, NCI-H446, NCI-H1299, NCI-H1650, H1792, NCI-H3255, A427, A549, 0225-02Sp, 2F7, 95-D, SPCA-1, LLC, Calu-1, Calu-3, L1022, PC9R, MSTO-211H and TKB-1, more preferably Calu-3 and A549;
preferably, the colon cancer cell is selected from the group consisting of HCT116, HCT116/FU, HCT-8/FU, LoVo, LoVo ADR, SW480, SW620, CaCo-2, RKO-E6, RKO-AS45-1, FET, HT55, HT115, HT-29, COLO 205, KM12, CL-40, KM12-SM, COLO320DM, NCI-H508, SW1417, COLO394 and WiDr, more preferably Caco-2.

3. The tumor cell-derived microparticle according to claim 1, the average particle size of the cellular vesicle is 200 nm to 800 nm, preferably 300 nm, 400 nm, 500 nm, 600 nm, 700 nm.

4. A method for preparing a tumor cell-derived microparticle, comprising the following steps:
1) providing a tumor cell expressing a spike protein binding receptor;
2) subjecting the tumor cell of step 1) to apoptosis;
preferably, the tumor cell of step 1) is subjected to apoptosis by means selected from the group consisting of ultraviolet irradiation, X-ray irradiation and chemotherapy drug;
3) collecting the cellular vesicle released by the apoptotic tumor cell; preferably collecting the cellular vesicle released by the apoptotic tumor cell by centrifugation, the average particle size of the cellular vesicle is 200 nm to 800 nm, preferably 300 nm, 400 nm, 500 nm, 600 nm, 700 nm.

5. The method according to claim 4, wherein:
the spike protein is a pneumonia virus spike protein;
the pneumonia virus is selected from the group consisting of SARS-CoV, SARS-CoV-2, and variants thereof;
preferably, the spike protein binding receptor is ACE2 or a binding fragment thereof;
the tumor cell is selected from the group consisting of a lung cancer cell and a colon cancer cell;
the lung cancer cell is selected from the group consisting of NCI-H196, NCI-H292, NCI-H460, NCI-H446, NCI-H1299, NCI-H1650, H1792, NCI-H3255, A427, A549, 0225-02Sp, 2F7, 95-D, SPCA-1, LLC, Calu-1, Calu-3, L1022, PC9R, MSTO-211H and TKB-1, more preferably Calu-3 and A549;
preferably, the colon cancer cell is selected from the group consisting of HCT116, HCT116/FU, HCT-8/FU, LoVo, LoVo ADR, SW480, SW620, CaCo-2, RKO-E6, RKO-AS45-1, FET, HT55, HT115, HT-29, COLO 205, KM12, CL-40, KM12-SM, COLO320DM, NCI-H508, SW1417, COLO394 and WiDr, more preferably Caco-2.

6. The method according to claim 4, wherein in step 3), the cellular vesicle is collected by a means comprising the following steps:
centrifuging the apoptotic tumor cell obtained in step 2) at 2 to 8°C at 800 to 1,000 g for 5 to 15 minutes to remove intact cell and obtain a first product;
centrifuging the first product at 2 to 8°C at 12,000 to 15,000 g for 1 to 3 minutes to remove cell debris and obtain a second product;
centrifuging the second product at 2 to 8°C at 12,000 to 15,000 g for 40 to 80 minutes to collect the released cellular vesicle.

7. A tumor cell-derived microparticle prepared by the method according to any one of claims 4 to 6.

8. Use of the tumor cell-derived microparticle according to any one of claims 1 to 3 and 7 in the preparation a medicament for treating viral pneumonia;
the virus is selected from the group consisting of SARS-CoV, SARS-CoV-2 and variants thereof.

9. A pharmaceutical composition comprising:
a pharmaceutically acceptable carrier; and
the tumor cell-derived microparticle according to any one of claims 1 to 3 and 7;
the pharmaceutical composition is prepared into a dosage form for lung administration.
